(19) **Europäisches Patentamt**
**European Patent Office**
**Office européen des brevets**

(11) **EP 1 354 137 B1**

(12) **EUROPÄISCHE PATENTSCHRIFT**

(45) Veröffentlichungstag und Bekanntmachung des Hinweises auf die Patenterteilung:
**13.06.2007 Patentblatt 2007/24**

(51) Int Cl.:
**F04D 13/06** (2006.01)

(21) Anmeldenummer: **02750864.7**

(22) Anmeldetag: **26.04.2002**

(86) Internationale Anmeldenummer:
**PCT/EP2002/004688**

(87) Internationale Veröffentlichungsnummer:
**WO 2002/088547 (07.11.2002 Gazette 2002/45)**

(54) **VERFAHREN ZUR REGELUNG EINER UNTERSTÜTZUNGSPUMPE FÜR FLUIDFÖRDERSYSTEME MIT PULSATILEM DRUCK**

METHOD FOR CONTROLLING AN ASSIST PUMP FOR FLUID DELIVERY SYSTEMS WITH PULSATILE PRESSURE

PROCEDE POUR REGULER UNE POMPE D'ASSISTANCE POUR DES SYSTEMES DE REFOULEMENT DE FLUIDE A PRESSION PULSEE

(84) Benannte Vertragsstaaten:
**AT CH FR GB IT LI**

(30) Priorität: **30.04.2001 DE 10123139**

(43) Veröffentlichungstag der Anmeldung:
**22.10.2003 Patentblatt 2003/43**

(73) Patentinhaber: **Berlin Heart AG**
**12247 Berlin (DE)**

(72) Erfinder:
• **NÜSSER, Peter**
**13051 Berlin (DE)**
• **MÜLLER, Johannes**
**10717 Berlin (DE)**
• **DEUS, Frank**
**12683 Berlin (DE)**
• **GÖTTEL, Peter**
**10435 Berlin (DE)**

• **HOFFMANN, Jan**
**12207 Berlin (DE)**
• **GRAICHEN, Kurt**
**13189 Berlin (DE)**
• **ARNDT, Andreas**
**12489 Berlin (DE)**
• **MERKEL, Tobias**
**14532 Kleinmachnow (DE)**

(74) Vertreter: **Pfenning, Meinig & Partner GbR**
**Patent- und Rechtsanwälte**
**Joachimstaler Strasse 10-12**
**10719 Berlin (DE)**

(56) Entgegenhaltungen:
**EP-A- 1 070 511       WO-A-00/64030**
**DE-A- 19 919 625      US-A- 4 598 697**
**US-A- 4 782 817       US-A- 4 846 152**
**US-A- 6 129 660**

**Beschreibung**

Die Erfindung betrifft ein Verfahren zur Regelung einer Unterstützungspumpe für Fluidfördersysteme mit pulsatilem Druck.

Es ist bekannt, zur Unterstützung von pulsatil arbeitenden Pumpen in einem Fluidfördersystem weitere Pumpen anzuordnen. Derartige Unterstützungspumpen werden üblicherweise mit konstanter Drehzahl angetrieben. Entsprechend der Differenzdruck/Volumenstrom-Kennlinie der Unterstützungspumpe reagiert diese bei Erhöhung des Eingangsdruckes und der damit verbundenen Änderung der Förderhöhe (Differenz vom Eingangs- zum Ausgangsdruck) mit einer Erhöhung des Volumenstromes und umgekehrt. Der Volumenstrom durch die Unterstützungspumpe sinkt jedoch auch in der Niedrigdruckphase, also bei abgesenktem Eingangsdruck, nicht auf null.

Je steiler die Differenzdruck/Volumenstrom-Kennlinie der Pumpe ist, desto höher ist der verbleibende Volumenstrom in dieser Phase. Das kann dazu führen, dass der Volumenstrom abreißt und das vor der Unterstützungspumpe liegende Fördersystem einschließlich der Hauptpumpe mit Unterdruck beaufschlagt wird, was zu verschiedenen Nachteilen führen kann. Z. B. kann das nachfließende Fluid beim Einfließen in die Pumpenkammer der Hauptpumpe stark verwirbelt werden.

Ein besonders empfindliches Fluidfördersystem stellt der Blutkreislauf dar. Blut zirkuliert, angetrieben durch rhythmische Kontraktionen des Herzens, in einem geschlossenen Gefäßsystem. Bei Störungen der Funktion des Herzens werden in jüngster Zeit Kreislaufunterstützungspumpen eingesetzt, die einen noch vorhandenen Puls des Herzens unterstützen sollen. Das Blut wird aus der linken Herzkammer unter Umgehung der Herzklappe in die Unterstützungspumpe und von dort aus in die Aorta geleitet. Derartige Unterstützungspumpen können sowohl nach dem Verdrängerprinzip als pulsatile Pumpen oder auch dem Turboprinzip als radiale oder axiale Strömungsmaschinen ausgeführt werden. Pulsatile Pumpen nach dem Verdrängerprinzip haben sich wegen des nötigen Aufwandes zur Synchronisierung mit dem Herzschlag nicht bewährt. Bei den Pumpen, die nach dem Turboprinzip arbeiten, werden die axialen Pumpen wegen ihrer geringeren Abmessungen bevorzugt.

Die bekannten axialen Blutpumpen bestehen im wesentlichen aus einem äußeren zylindrischen Rohr, in dem ein Förderteil, das als Rotor eines außen anliegenden Motorstators ausgebildet ist, rotiert und das Blut in axialer Richtung bewegt. Ebenfalls bekannt ist es, den Rotor berührungsfrei magnetisch zu lagern. Eine derartige Unterstützungspumpe ist aus WO 00/640 30 bekannt.

Wird eine solche Unterstützungspumpe mit konstanter Drehzahl betrieben, kommt es aufgrund der oben beschriebenen Zusammenhänge dazu, dass das Blut durch die Unterstützungspumpe weiter gefördert wird, auch wenn die Herzkammer sich in der Dekontraktionsphase befindet.

Der Erfindung liegt die Aufgabe zu Grunde, ein Verfahren anzugeben, mit dem der Volumenstrom einer Unterstützungspumpe auf einfacher Weise nur in der Druckphase der Hauptpumpe unterstützend wirkt.

Erfindungsgemäß wird die Aufgabe gelöst durch die Merkmale des Anspruches 1. Zweckmäßige Ausgestaltungen sind Gegenstand der Unteransprüche.

Danach werden ständig die Druckdifferenz zwischen Ein- und Ausgangsseite der Unterstützungspumpe sowie die Durchflussmenge durch die Unterstützungspumpe ermittelt. Die Drehzahl der Unterstützungspumpe wird so geregelt, dass die ermittelte Druckdifferenz nicht unter einen vorbestimmbaren Wert fällt und die Durchflussmenge nicht unter null sinkt.

Nach einer bevorzugten Ausführungsform des Verfahrens wird als Unterstützungspumpe eine Axialpumpe mit elektronisch kommutiertem Synchronmotor und permanentmagnetischer Lagerung und Steuerspulen zur magnetischen Lageregelung des Rotors benutzt und die Druckdifferenz zwischen Ein- und Ausgangsseite der Unterstützungspumpe ermittelt, indem aus dem Steuerstrom der Steuerspulen und der aktuellen Rotorposition, die als Werte der Lageregelung vorliegen, die der Druckdifferenz proportionale Störkraft auf den Rotor bestimmt wird. Gleichzeitig lässt sich die Durchflussmenge durch die Unterstützungspumpe anhand der aktuellen Drehzahl und der Druckdifferenz aus dem zuvor gemessenen Differenzdruck/Volumenstrom-Kennfeld der Unterstützungspumpe ermitteln. Auf diese Weise werden keinerlei separate Sensoren für Druck und Durchfluss benötigt.

Die Erfindung soll nachstehend anhand einer Kreislaufunterstützungspumpe als Ausführungsbeispiel für das Verfahren näher erläutert werden. In den Zeichnungen zeigen:

Fig. 1     ein Kennfeld einer Axialpumpe,

Fig. 2     eine für die Durchführung des Verfahrens geeignete Herzunterstützungspumpe,

Fig. 3     ein Schema eines Beispiels für die erfindungsgemäße Regelung,

Fig. 4     den Druckverlauf an der Herzunterstützungspumpe,

Fig. 5    den Förderhöhe- und Volumenstromverlauf an der Herzunterstützungspumpe,

Fig. 6    den Drehzahlverlauf der Herzunterstütungspumpe,

Fig. 7    dagegen den Förderhöhe- und Volumenstromverlauf und

Fig. 8    den Druckverlauf an einer Herzunterstützungspumpe beim Betrieb mit konstanter Drehzahl.

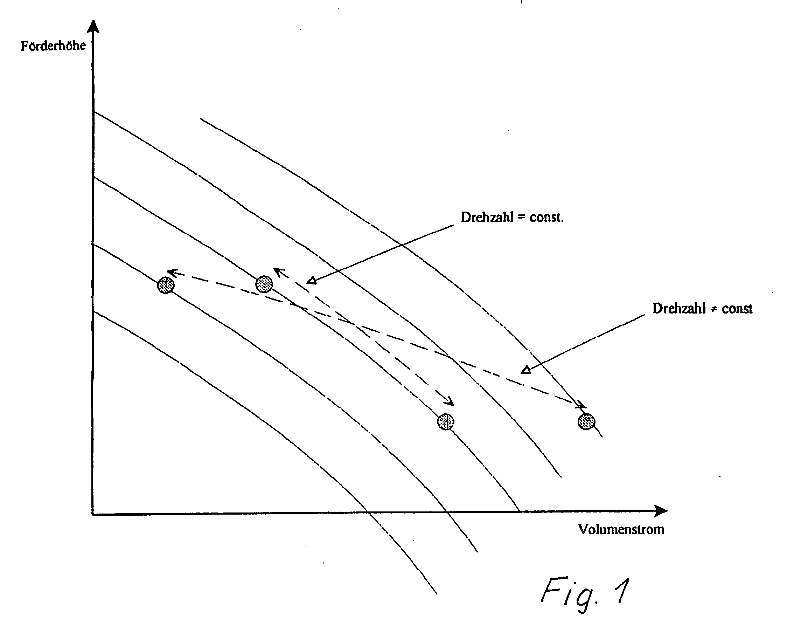

Fig. 1 zeigt ein Kennfeld einer Blutpumpe mit axialer Strömung. Die Kennlinien zeigen jeweils die Abhängigkeit des Drucksprungs der Pumpe (Förderhöhe) vom geförderten Volumen pro Zeiteinheit bei einer bestimmten Drehzahl. Bei einem Betrieb mit konstanter Drehzahl bewegt sich der Arbeitspunkt der Pumpe also entlang einer Kennlinie. Das Ausmaß der Flussänderung bei gegebener Druckdifferenzänderung hängt von der Steilheit der Kennlinie ab. Um diesen Wert scheinbar zu verändern, muss die Drehzahl der Pumpe in Abhängigkeit von der momentanen Förderhöhe verändert werden. Der Arbeitspunkt bewegt sich jetzt nicht mehr längs einer Linie konstanter Drehzahl, sondern wandert zwischen Linien unterschiedlicher Drehzahl. Die Drehzahl ist dabei der zu beeinflussende Parameter. Soll eine gegebene Vergrößerung der Förderhöhe eine größere Verringerung des Flusses verursachen als durch die natürliche Pumpenkennlinie vorgegeben, dann muss die Rotordrehzahl bei steigender Förderhöhe abgesenkt und bei fallender Förderhöhe angehoben werden. Daraus resultiert eine Wanderung im Kennfeld und somit eine scheinbare Verringerung der Steilheit der Pumpenkennlinie.

Für ein natürliches Blutkreislaufsystem gilt außerdem die Bedingung, dass das Blut während der diastolischen Phase nicht wieder an das Herz zurückfließen soll. Bei einem intakten Kreislaufsystem übernimmt diese Funktion eine Herzklappe. Die Blutpumpe muss deshalb auch die Funktion der Herzklappe nachbilden. Für die erfindungsgemäße Regelung müssen deshalb die Förderhöhe und der Volumenstrom bekannt sein. Diese können mit einer geeigneten Sensorik ermittelt werden. Sie können jedoch bei geschickter Wahl einer bestimmten Pumpenart auch aus Regeldaten des Pumpenantriebes selbst gewonnen werden.

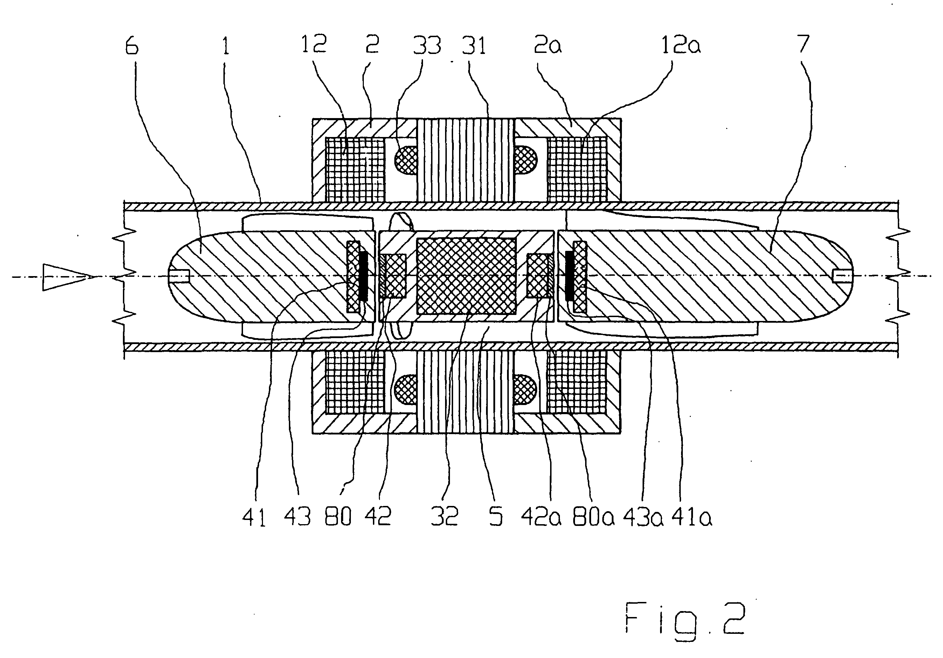

Fig. 2 zeigt eine solche zur Durchführung des Verfahrens geeignete axiale Blutunterstützungsumpe. Der Antrieb der Blutunterstützungspumpe arbeitet nach dem Prinzip eines elektronisch kommutierten Synchronmotors. Der Motor hat einen Stator, bestehend aus einem Blechpaket 31, Wicklungen 33 und Eisenrückschlußkappen 2, 2a, und einen Rotor 5 mit permanentmagnetischem Kern 32. Der Stator umschließt einen rohrförmigen Hohlkörper 1, in dem in axialer Richtung ein Fluid, im vorliegenden Falle also Blut, gefördert wird. Der Rotor 5 ist berührungsfrei magnetisch gelagert.

Das magnetische Lager besteht aus Permanentmagneten 42, 42a an den Rotorstirnseiten und Permanentmagneten 41, 41a an den Stirnseiten von Leiteinrichtungen 6 und 7. Die Leiteinrichtungen 6, 7 sind an der Innenwand des rohrförmigen Hohlkörpers 1 befestigt.

Zum magnetischen Lager gehören außerdem Steuerspulen 12, 12a. Zur Messung der aktuellen Rotorposition dienen Sensorspulen 43, 43a in den Leiteinrichtungen 6, 7 und diesen gegenüberliegende Kurzschlußringe 80, 80a.

Die Paare der Permanentmagneten 41, 42; 41a, 42a sind jeweils auf Anziehung gepolt. Magnetisch liegen die Paare in Reihe.

Ohne eine zusätzliche Stabilisierung würde der Rotor 5 zu einer Seite hin angezogen werden, es besteht in axialer Richtung ein instabiles Gleichgewicht. In radialer Richtung wirken beide Magnetpaare zentrierend, die radiale Lage ist somit passiv stabil.

Die Steuerspulen 12, 12a sind elektrisch in Reihe geschaltet und magnetisch so angeordnet, dass ein Strom das Magnetfeld des einen Magnetpaares schwächt und das des anderen verstärkt. Der magnetische Rückschluss geschieht über die Eisenrückschlußkappen 2, 2a und das Blechpaket 31 des Stators.

Die axiale Position des Rotors 5 kann mit Hilfe der Sensorspulen 43, 43a ermittelt werden. Die Sensorspulen 43, 43a werden mit einer höherfrequenten Spannung beaufschlagt. Bei axialer Bewegung des Rotors 5 kommt es zu einer gegenseitigen verstimmung der Sensorspulen 43, 43a. Durch Anordnung der Sensorspulen 43, 43a in einer Brückenschaltung lässt sich ein Messsignal für die axiale Position des Rotors 5 gewinnen.

Die axiale Stabilisierung erfolgt über einen Regelkreis. Die gemessene Rotorposition ist das Eingangssignal des Reglers. Dessen Ausgangssignal, der Stellstrom, wird in die Steuerspulen 12, 12a eingespeist. Auf diese Weise lässt sich die Position des Rotors 5 zwischen den beiden Endanschlägen regeln. Die Regelung wird dann stromlos, wenn die Summe aller magnetischen und mechanischen Kräfte null ist. Bei unbelastetem Motor ist dies in der Mittellage der Fall. Dort ist der Steuerstrom praktisch unendlich klein. Wenn der Rotor 5 axial belastet wird, muss er der angreifenden Kraft entgegen verschoben werden, bis die dann asymmetrischen Kräfte der Permanentmagneten 41, 42; 41a, 42a die Störkraft kompensieren. An diesem Punkt ist der Steuerstrom wieder unendlich klein.

Der Regler ist als PID-Regler mit $I_2$-Anteil zur Nullstromregelung ausgeführt. Der Regler kann sprungförmige Störungen nahezu ohne Überschwingen ausregeln. Die Nullpunktsuche ist schnell genug, um bei der anwendungsspezifischen Störfrequenz die Steuerstromaufnahme nahe null zu halten.

Das Messsignal wird aus einer Brückenschaltung der Sensorspulen 43, 43a gewonnen. Die Messung wird allerdings durch die Regelung des Steuerspulenstromes und des Motorstromes erschwert. Mit einem Ausblendverfahren werden Messungen deshalb nur zu störfreien Zeiten zwischen den Schaltimpulsen vorgenommen. Während der Schaltimpulsdauer wird der letzte Messwert vor dem Ausblenden gespeichert.

Die axiale Stabilisierung des magnetisch gelagerten Rotors 5 gestattet die Schätzung der auf den Rotor 5 wirkenden Störkraft. Die Summe der auf den Rotor 5 wirkenden Kräfte muss zu jedem Zeitpunkt gleich null sein. Kräfte des Permanentmagnetsystems, Kräfte des Elektromagnetsystems und mechanische Kräfte, insbesondere Druckkräfte, sowie Reibungs-, Dämpfungs- und Beschleunigungskräfte müssen einander aufheben. Unter der Voraussetzung,- dass die Frequenzen der zu erfassenden Störkräfte gering im Vergleich zur Grenzfrequenz des Stabilisierungsregelkreises sind, können die Dämpfungs- und Beschleunigungskräfte für die Berechnung vernachlässigt werden. Somit errechnet sich die Störkraft wie folgt:

$$\texttt{Störkraft = Steuerstrom x elektrische Empfindlichkeit -}$$
$$\texttt{Rotorposition x Axialsteifigkeit.}$$

Die elektromagnetische Empfindlichkeit ist eine von dem magnetischen Kreis abhängige Konstante. Die Axialsteifigkeit ist ein Ausdruck für die Kraft, die nötig ist, um den Rotor 5 um einen bestimmten Weg axial zu verschieben und ist in dem hier interessierenden Bereich (Rotorspalt ca. 0,5 bis 2,5 mm) ebenfalls konstant.

Mit der Störkraft steht ein Wert für den proportionalen Drucksprung der Unterstützungspumpe zur Verfügung, der sich als dynamisches Signal für die Regelung der Drehzahl nutzen lässt. Gleichzeitig lässt sich mit Hilfe der Druckdifferenz und der Pumpendrehzahl bei bekannter Pumpenkennlinie der Volumenstrom berechnen.

Durch die Wahl einer speziellen Blutpumpe und die geschickte Verwertung der von der Rotorlageregelung her bekannten Daten gelingt es so, auf Sensoren für Druck und Durchfluss völlig zu verzichten.

Die hier gezeigte Blutpumpe ist auch aus anderen Gründen für die erfindungsgemäße Regelung besonders geeignet. Der sensorlos kommutierte Synchronmotor gestattet eine hohe Winkelbeschleunigung des Pumpenrotors. Diese Beschleunigung und die damit verbundenen Axial- und Radialkräfte auf den Rotor 5 werden von der magnetischen Lagerung toleriert. Der nutzbare Drehzahlbereich wird nicht von Resonanzfrequenzen der radialen Rotoraufhängung begrenzt. Resonanzen bleiben immer gedämpft. Folglich ist eine Drehzahlvariation von minimaler Drehzahl bis maximaler Drehzahl in einer Zeit von ca. 50 ms möglich. Zwischen der Zeitfunktion der Rotordrehzahl und der des Pumpenflusses sind keine Verzögerungszeiten erkennbar.

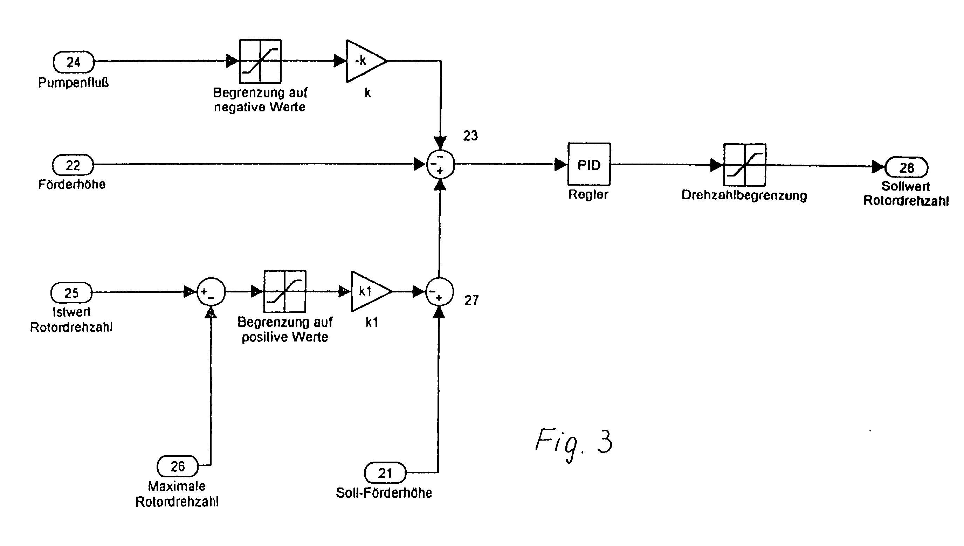

Fig. 3 zeigt ein Beispiel für einen möglichen Regelkreis für die Drehzahlregelung. Die Soll-Förderhöhe der Unterstützungspumpe wird am Zweig 21 eingegeben. Sie richtet sich danach, welcher natürliche Druck durch das Herz noch erzeugt werden kann und wieviel zusätzlicher Druck dementsprechend von der Unterstützungspumpe aufgebracht werden soll. Die wie zuvor beschrieben ermittelte Druckdifferenz zwischen Ein- und Ausgangsseite der Unterstützungspumpe wird als nötige Förderhöhe am Zweig 22 zu einem Soll-Ist-Vergleich an einen Vergleicher 23 eingegeben. Hieraus ergibt sich die Regelabweichung, die von einem PID-Regler mit nachfolgendem Begrenzer für den Stellwert der Drehzahl ausgeregelt wird. Dieser Stellwert wird von der Motorsteuerung in eine entsprechende Drehzahl umgesetzt.

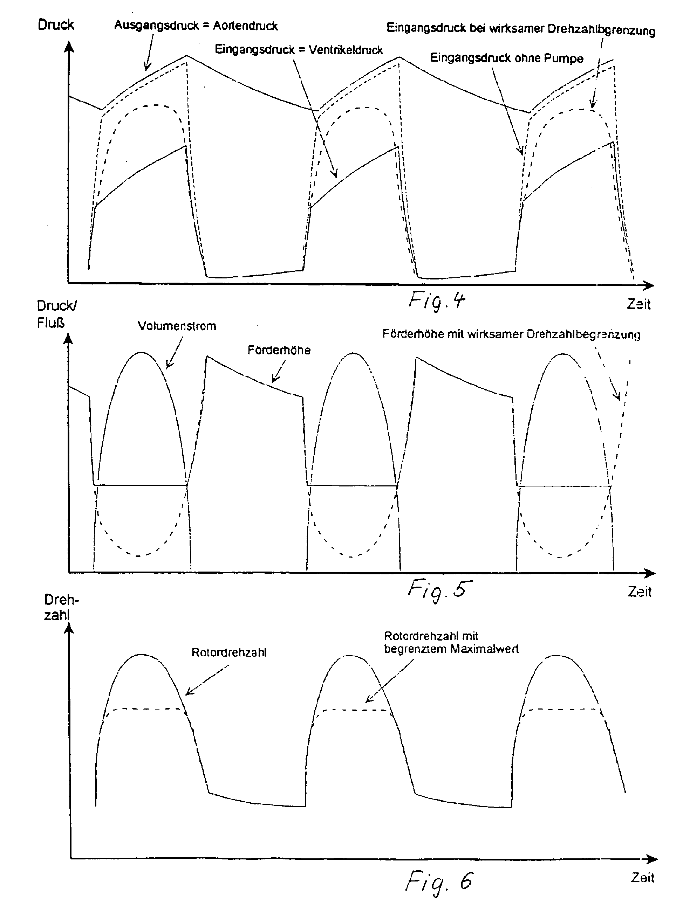

Die Fig. 4 bis 6 zeigen die Zeitverläufe von charakteristischen Daten in der systolischen und diastolischen Phase. Die Unterstützungspumpe befindet sich zwischen einer Ausleitung aus der linken Herzkammer (Ventrikel) und der Aorta. Der Ventrikeldruck des Herzens bildet den Eingangsdruck der Unterstützungspumpe, der Aortendruck durch die Unterstützungspumpe ist gleichzeitig deren Ausgangsdruck.

Die Druckdifferenz zwischen dem natürlichen Druck des Ventrikels und dem angestrebten Aortendruck ist in der systolischen Phase von der Unterstützungspumpe aufzubringen. In der diastolischen Phase soll lediglich ein Rückfluss vom Blut zur Herzkammer verhindert werden.

Fig. 5 zeigt die sich aus diesen Bedingungen ergebende Förderhöhe der Unterstützungspumpe. Der Sollwert der Förderhöhe entspricht etwa dem halben mittleren Aortendruck. Wird dieser Sollwert unterschritten, setzt die Drehzahlregelung ein, die Pumpe beschleunigt dann ggf. bis zu einer maximalen Drehzahl und fördert das Blut in die Aorta. Steigt die Förderhöhe bei nachlassendem Druck des Ventrikels wieder an, sinkt der Drehzahl-Stellwert und damit die Rotordrehzahl bis zum Erreichen der diastolischen Phase.

Durch Aufschalten des Volumenstroms am Zweig 24, und zwar nur der Negativ-Werte, das heißt einem eventuellen Rückfluss zum Ventrikel, über einen Regelverstärker k auf den Vergleicher 23 wird gewährleistet, dass in der diastolischen Phase eine solche Drehzahl aufrecht erhalten wird, dass der Volumenstrom nicht unter null sinkt, sondern auf einem Wert bei null gehalten wird.

Im vorliegenden Ausführungsbeispiel wird die Soll-Förderhöhe außerdem mit einem Korrekturwert beaufschlagt, der sich aus dem Vergleich der aktuellen Rotordrehzahl mit einer maximalen Rotordrehzahl ergibt. Steigt die am Zweig 25 anliegende Rotordrehzahl über eine am Zweig 26 vorgegebene maximale Rotordrehzahl, wird die Abweichung, über einen Regelverstärker k1 verstärkt, mit negativem Vorzeichen einem Vergleicher 27 zugeführt, an dem die Sollförderhöhe anliegt. Die auszuregelnde Soll-Ist-Abweichung der Förderhöhe wird bei Erreichen einer Maximal-Rotordrehzahl somit von vornherein begrenzt.

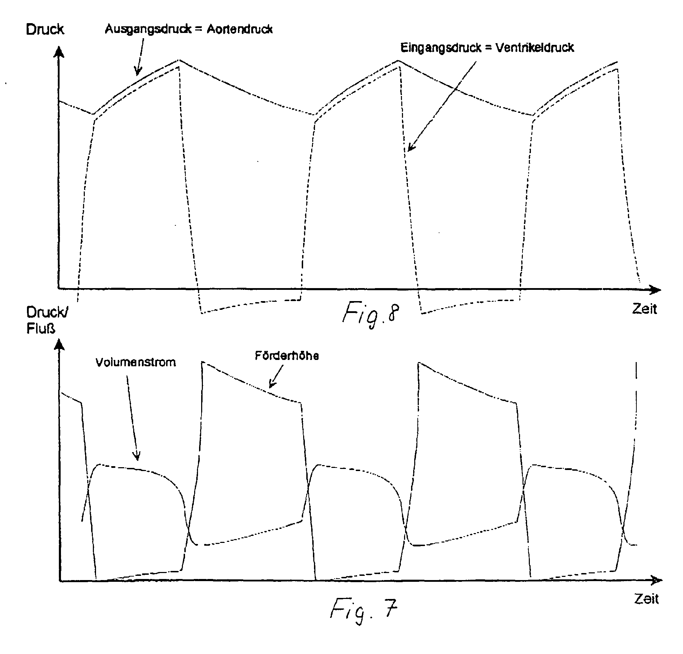

Fig. 7 zeigt dagegen den Verlauf der Förderhöhe und des Volumenstromes und Fig. 8 den Druckverlauf am Ventrikel beim Betrieb einer Pumpe mit konstanter Drehzahl. Es ist erkennbar, dass die Pumpe auch in der diastolischen Phase saugt und die Herzkammer leer pumpt. Es kommt nicht zu der gewünschten Druckentlastung des Herzens.

Mit dem in diesem Ausführungsbeispiel gezeigten Verfahren kann nicht nur auf störende und zusätzlichen Aufwand verursachende Sensorik verzichtet werden, sondern auch ein in einem Fluidfördersystem herrschender pulsatiler Volumenstrom unterstützt werden, ohne dass mittels Maßnahmen zur Synchronisation zwischen den beiden Betriebszuständen unterschieden werden muss.

**Bezugszeichenliste**

| | |
|---|---|
| 2 | Eisenrückschlußkappe |
| 2a | Eisenrückschlußkappe |
| 5 | Rotor |
| 6 | Leiteinrichtung |
| 7 | Leiteinrichtung |
| 12 | Steuerspule |
| 12a | Steuerspule |
| 21 | Zweig |
| 22 | Zweig |
| 23 | Vergleicher |
| 24 | Zweig |
| 25 | Zweig |
| 26 | Zweig |
| 27 | Vergleicher |
| 31 | Blechpaket |
| 33 | Wicklungen |
| 41 | Permanentmagnet |
| 41a | Permanentmagnet |
| 42 | Permanentmagnet |
| 42a | Permanentmagnet |
| 43 | Sensorspule |
| 43a | Sensorspule |
| k | Regelverstärker |
| k1 | Regelverstärker |
| 80 | Kurzschlussring |
| 80a | Kurzschlussring |

**Patentansprüche**

1. Verfahren zur Regelung einer Unterstützungspumpe eines Fluidfördersystemes mit einer Hauptpumpe mit pulsatilem Druck,
**dadurch gekennzeichnet, dass**
ständig die Druckdifferenz zwischen Ein- und Ausgangsseite der Unterstützungspumpe sowie die Durchflussmenge durch die Unterstützungspumpe ermittelt werden und die Drehzahl der Unterstützungspumpe so geregelt wird, dass die.ermittelte Druckdifferenz nicht unter einen vorbestimmbaren Wert fällt und die Durchflussmenge nicht unter null sinkt.

2. Verfahren nach Anspruch 1,
**dadurch gekennzeichnet, dass**
als Unterstützungspumpe eine Axialpumpe mit elektronisch kommutiertem Synchronmotor und permanentmagnetischer Lagerung und Steuerspulen zur magnetischen Lageregelung des Rotors benutzt wird und die Druckdifferenz zwischen Ein- und Ausgangsseite der Unterstützungspumpe ermittelt wird, indem aus dem Steuerstrom der Steuerspulen und der aktuellen Rotorposition, die als Werte der Lageregelung vorliegen, die der Druckdifferenz proportionale Störkraft auf den Rotor bestimmt wird.

3. Verfahren nach Anspruch 1 oder 2,
**dadurch gekennzeichnet, dass**
die Durchflussmenge durch die Unterstützungspumpe anhand der aktuellen Drehzahl und der Druckdifferenz aus dem zuvor gemessenen Differenzdruck/Volumenstrom-Kennfeld der Unterstützungspumpe ermittelt wird.

4. Verfahren nach einem der vorhergehenden Ansprüche,
**dadurch gekennzeichnet, dass**
die Drehzahl der Unterstützungspumpe auf einen vorbestimmbaren Maximalwert begrenzt wird.

5. Verfahren nach einem der vorhergehenden Ansprüche,
**dadurch gekennzeichnet, dass**
zur Regelung der Drehzahl ein PID-Regler verwendet wird.

6. Verfahren nach einem der vorhergehenden Ansprüche,
**dadurch gekennzeichnet, dass**
dem Druckdifferenz-Sollwert ein aus einem Vergleich aus der Rotordrehzahl und einem vorbestimmbaren Maximalwert der Rotordrehzahl ermittelter Korrekturwert aufgeschaltet wird.


**Claims**

1. Method for controlling an assist pump for fluid delivery systems having a main pump that delivers pulsatile pressure,
**characterised in that**
the pressure difference between the inflow side and the outflow side of the assist pump and the volume flow rate through the assist pump are measured continuously and the rotational speed of the assist pump is controlled in such a way that the determined pressure difference does not drop below a predetermined value and the volume flow rate does not drop below zero.

2. Method according to claim 1,
**characterised in that**
an axial pump with an electronically commutated synchronous motor and permanent-magnetic bearings and control coils for the magnetic control of the position of the rotor is used as an assist pump, and that the pressure difference between the inflow side and the outflow side of the assist pump is determined from the disturbance force which is proportional to this pressure difference acting on the rotor and which, in turn, is calculated on the basis of the control current through the control coils and the actual rotor position which are values known from the position control mechanism.

3. Method according to claim 1 or 2,
**characterised in that**
the volume flow rate through the assist pump is determined on the basis of the actual rotational speed, the pressure difference and the previously determined differential pressure / volume flow rate - characteristic graph field of the assist pump.

4. Method according to one of the preceding claims,
**characterised in that**
the rotational speed of the assist pump is limited to a predetermined maximum value.

5. Method according to one of the preceding claims,
**characterised in that**
a PID-controller is used to control the rotational speed.

6. Method according to one of the preceding claims,
**characterised in that**
a correction value determined by comparing the rotational speed of the rotor to a predetermined maximum value of the rotational speed of the rotor is added to the pressure difference set-point value.

**Revendications**

1.  Procédé de réglage d'une pompe d'assistance d'un système de refoulement de fluide comportant une pompe principale par une pression pulsatile, **caractérisé en ce que** la différence de pression entre le côté d'admission et le côté d'échappement de la pompe d'assistance, ainsi que le débit à travers la pompe d'assistance sont déterminées en continu et la vitesse de rotation de la pompe d'assistance est réglée de telle sorte que la différence de pression déterminée ne chute pas en dessous d'une valeur prédéfinissable et que le débit ne chute pas en dessous de zéro.

2.  Procédé selon la revendication 1, **caractérisé en ce que** la pompe d'assistance utilisée est une pompe axiale avec un moteur synchrone, commuté par voie électronique, et un palier à aimant permanent et des bobines de commande pour le réglage du palier magnétique du rotor, et **en ce que** la différence de pression entre le côté d'admission et le côté d'échappement de la pompe d'assistance est déterminée du fait qu'à partir du courant de commande des bobines de commande et à partir de la position actuelle du rotor, qui sont disponibles en tant que valeurs de réglage du palier, est déterminée la force de perturbation exercée sur le rotor qui est proportionnelle à la différence de pression.

3.  Procédé selon la revendication 1 ou 2, **caractérisé en ce que** le débit à travers la pompe d'assistance est déterminé au moyen de la vitesse de rotation actuelle et de la différence de pression à partir de la caractéristique de fonctionnement en pression différentielle et flux volumétrique de la pompe d'assistance, mesurée au préalable.

4.  Procédé selon l'une quelconque des revendications précédentes, **caractérisé en ce que** la vitesse de rotation de la pompe d'assistance est limitée à une valeur maximale prédéfinissable.

5.  Procédé selon l'une quelconque des revendications précédentes, **caractérisé en ce qu'**un régulateur PID est utilisé pour le réglage de la vitesse de rotation.

6.  Procédé selon l'une quelconque des revendications précédentes, **caractérisé en ce qu'**à la valeur de consigne de la différence de pression est ajoutée une valeur de correction déterminée par comparaison entre la vitesse de rotation du rotor et une valeur maximale prédéfinissable pour la vitesse de rotation du rotor.

EP 1 354 137 B1

Förderhöhe

Drehzahl = const.

Drehzahl ≠ const

Volumenstrom

*Fig. 1*

6   1   12   2   33   31   2a   12a   7

41  43  80  42   32   5   42a  80a  43a  41a

Fig. 2

EP 1 354 137 B1

Sollwert
Rotordrehzahl

28

Drehzahlbegrenzung

PID

Regler

23

27

-k

k

k1

k1

Begrenzung auf
negative Werte

Begrenzung auf
positive Werte

21

Soll-Förderhöhe

26

Maximale
Rotordrehzahl

24

Pumpenfluß

22

Förderhöhe

25

Istwert
Rotordrehzahl

*Fig. 3*

Druck

Ausgangsdruck = Aortendruck

Eingangsdruck = Ventrikeldruck

Eingangsdruck bei wirksamer Drehzahlbgrenzung

Eingangsdruck ohne Pumpe

*Fig. 4*

Zeit

Druck/ Fluß

Volumenstrom

Förderhöhe

Förderhöhe mit wirksamer Drehzahlbegrenzung

*Fig. 5*

Zeit

Drehzahl

Rotordrehzahl

Rotordrehzahl mit begrenztem Maximalwert

*Fig. 6*

Zeit

Druck

Ausgangsdruck = Aortendruck

Eingangsdruck = Ventrikeldruck

Zeit

Fig. 8

Druck/
Fluß

Volumenstrom

Förderhöhe

Zeit

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0064030 A